(19) Europäisches Patentamt
European Patent Office
Office européen des brevets

(11) EP 2 744 574 B1

(12) **EUROPÄISCHE PATENTSCHRIFT**

(45) Veröffentlichungstag und Bekanntmachung des Hinweises auf die Patenterteilung:
**18.10.2017 Patentblatt 2017/42**

(21) Anmeldenummer: **12738092.1**

(22) Anmeldetag: **17.07.2012**

(51) Int Cl.:
***A61Q 5/00*** (2006.01) ***A61K 8/73*** (2006.01)
***A61K 8/81*** (2006.01) ***A61K 8/04*** (2006.01)

(86) Internationale Anmeldenummer:
**PCT/EP2012/063942**

(87) Internationale Veröffentlichungsnummer:
**WO 2013/023855 (21.02.2013 Gazette 2013/08)**

# (54) VERWENDUNG EINES MITTELS FÜR KERATINHALTIGE FASERN, ENTHALTEND MINDESTENS EINE NICHTIONISCHE, MITTELS PROPYLENOXID MODIFIZIERTE STÄRKE UND MINDESTENS EIN ZUSÄTZLICHES FILMBILDENDES UND/ODER FESTIGENDES ZUR VERBESSERUNG DES FARBERHALTS OXIDATIVER HAARCOLORATIONEN

USE OF AN AGENT FOR KERATIN FIBERS, CONTAINING AT LEAST ONE NONIONIC STARCH MODIFIED BY MEANS OF PROPYLENE OXIDE AND AT LEAST ONE ADDITIONAL FILM-FORMING AND/OR STRENGTHENING POLYMER, FOR IMPROVING THE COLOR PRESERVATION OXIDATIVE HAIR COLORATIONS

UTILISATION D'UN AGENT POUR FIBRES KÉRATINIQUES, CONTENANT AU MOINS UN AMIDON NON IONIQUE, MODIFIÉ PAR DE L'OXYDE DE PROPYLÈNE, ET AU MOINS UN AUTRE POLYMÈRE FILMOGÈNE ET/OU FIXATEUR POUR AMÉLIORER LA TENUE DE COLORATIONS OXYDATIVES DE CHEVEUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.08.2011 DE 102011081108**

(43) Veröffentlichungstag der Anmeldung:
**25.06.2014 Patentblatt 2014/26**

(73) Patentinhaber: **Henkel AG & Co. KGaA**
**40589 Düsseldorf (DE)**

(72) Erfinder:
- **METTEN, Diane**
**22393 Hamburg (DE)**
- **SCHEFFLER, Rene**
**25479 Ellerau (DE)**

(56) Entgegenhaltungen:
**EP-A1- 1 568 351** **WO-A2-2009/134555**
**FR-A1- 2 936 413** **FR-A1- 2 944 967**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 744 574 B1

## Beschreibung

Die vorliegende Erfindung betrifft die Verwendung eines Mittels zur Haarbehandlung, enthaltend eine Kombination mindestens einer nichtionischen, mittels Propylenoxid modifizierten Stärke mit mindestens einem filmbildenden und/oder festigenden anionischen Polymer, zur Verbesserung des Farberhalts oxidativer Haarcolorationen sowie entsprechende Verfahren zur Behandlung keratinhaltiger Fasern.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Die Färbung keratinischer Fasern, beispielsweise menschlicher Keratinfasern erfolgt in der Regel mit Färbezusammensetzungen, die neben weiteren Bestandteilen Oxidationsfarbstoffvorprodukte, insbesondere Oxidationsbasen ("Entwickler") enthalten. Diese farblosen oder schwach farbigen Substanzen reagieren nach Zugabe eines Oxidationsmittels im Rahmen einer oxidativen Kondensation zu Farbstoffmolekülen. Zur Nuancierung der so erhaltenen Farben wird den Entwicklern in der Regel eine zweite Gruppe von Oxidationsfarbstoffvorprodukte, die sogenannten Kuppler, zugesetzt. Durch die Kombination von Entwickler- und Kupplerkomponenten lässt sich eine Vielzahl unterschiedlicher Farbtöne realisieren.

In Ergänzung oder alternativ zu den vorgenannten Oxidationsfarbstoffen werden Keratinfasern mit Hilfe direktziehender Farbstoffe gefärbt. Bei diesen direktziehenden Farbstoffen handelt es sich um farbige Moleküle die auf die Oberfläche der Keratinfasern aufziehen.

Die durch Oxidationsfarbstoffe oder direktziehende Farbstoffe erhaltene Färbung der keratinischen Faser verblasst durch äußere Einflüsse wie Licht, insbesondere jedoch aufgrund wiederholter Haarwäsche.

Zur Verbesserung des Farberhalts oxidativ gefärbter Keratinfasern wird in den europäischen Patenten EP 1 915 981 B1 und EP 1 923 042 B1 beispielsweise der Einsatz von Zinksalzen vorgeschlagen. Die Zinksalze werden nach Lehre dieser Dokumente nach Beendigung des Färbevorgangs auf die keratinischen Fasern aufgebracht.

Weiterhin werden zur Verbesserung des Farberhalts in den Patentanmeldungen EP 1 568 351 A1, FR 2 944 967 A1 sowie WO 2009/1345855 A2 Amylose-haltige Stärken bzw. anionische oder nichtionische oxidierte Polysaccharide bzw. eine Mischung aus Silicon-Polyurethanpolymer, Ester und fluoriertem Silicon beschrieben. In der französischen Patentanmeldung FR 2 936 416 A1 wird der Einsatz von Silan-haltigen Vorbehandlungsmitteln offenbart.

Die nach Lehre dieser Dokumente erzielten Farberhaltungswerte sind jedoch nicht in jedem Falle zufriedenstellend.

Zusammenfassend besteht damit weiterhin ein Bedarf nach Verfahren zur Verbesserung des Farberhalts gefärbter keratinischer Fasern. Vor diesem Hintergrund wurde festgestellt, dass durch die Behandlung keratinischer Fasern mit einer Kombination spezieller Polymere ein verbesserter Farberhalt erreicht werden kann.

Ein erster Gegenstand der vorliegenden Erfindung ist daher die Verwendung eines Mittels, enthaltend in einem kosmetisch akzeptablen Träger

(a) mindestens eine nichtionische, mittels Propylenoxid modifizierte Stärke in einer Menge von 4,0 Gew.-% bis 15 Gew.-%, bezogen auf das Gewicht des Mittels, wobei die nichtionische, mittels Propylenoxid modifizierte Stärke ein mittleres Molekulargewicht (Gewichtsmittel) von 700 bis 1000 kDa aufweist und wobei die nichtionische, mittels Propylenoxid modifizierte Stärke einen Propylenoxidgehalt von 1 bis 20 Gew.-%, bezogen auf das Gewicht der modifizierten Stärke, aufweist,
und

(b) mindestens ein filmbildendes und/oder festigendes anionisches Polymer in einer Menge von 1,0 Gew.-% bis 9,0 Gew.-%, bezogen auf das Gewicht des Mittels, wobei das filmbildende und/oder festigende Polymer mindestens eine Struktureinheit der Formel (I) und mindestens eine Struktureinheit der Formel (II) umfasst,

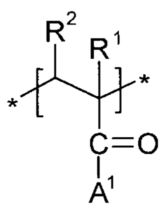 (I)

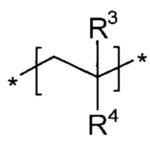 (II)

worin

$R^1$ und $R^2$ unabhängig voneinander für ein Wasserstoffatom oder eine Methylgruppe stehen, mit der Maßgabe, dass $R^1$ und $R^2$ nicht gleichzeitig für eine Methylgruppe stehen,
$R^3$ für ein Wasserstoffatom oder eine Methylgruppe steht,
$R^4$ für eine Carbamoyl-Gruppe, eine lineare oder verzweigte ($C_4$ bis $C_{12}$)-Alkylaminocarbonylgruppe, eine lineare oder verzweigte ($C_4$ bis $C_{12}$)-Alkylaminoethyl-aminocarbonylgruppe, eine lineare oder verzweigte ($C_4$ bis $C_{12}$)-Alkylaminopropyl-aminocarbonylgruppe, eine lineare oder verzweigte ($C_4$ bis $C_{12}$)-Alkyloxycarbonylgruppe, eine lineare oder verzweigte ($C_4$ bis $C_{12}$)-Alkylaminoethyloxycarbonylgruppe, eine lineare oder verzweigte ($C_4$ bis $C_{12}$)-Alkylaminopropyloxycarbonylgruppe, eine lineare oder verzweigte ($C_2$ bis $C_{12}$)-Acyloxygruppe steht,
$A^1$ für eine Hydroxygruppe oder einen organischen Rest mit mindestens einer Sulfonsäuregruppe steht, der über ein Sauerstoffatom oder eine Gruppe NH an das Strukturfragment bindet

zur Verbesserung des Farberhalts oxidativ gefärbter keratinhaltiger Fasern, insbesondere oxidativ gefärbten menschlichen Haars.

Die Verbesserung des Farberhalts umfasst erfindungsgemäß den Erhalt der Farbe bei Einwirkung äußerer Einflüsse. Der Farberhallt umfasst damit beispielsweise Phänomene wie die Waschechtheit, Lichtechtheit, Reibechtheit oder Schweißechtheit der oxidativen Haarfärbung. Besondere Vorteile weist die erfindungsgemäße Verwendung im Hinblick auf den Farberhalt nach Wäsche der Keratinfasern auf. Die Verwendung nach erfindungsgemäßer Zusammensetzungen zur Verbesserung der Waschechtheit oxidativ gefärbter keratinhaltiger Fasern, insbesondere oxidativ gefärbten menschlichen Haars, ist daher eine bevorzugte Ausführungsform der vorliegenden Anmeldung.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers werden häufig der so genannte Drei-Punkt-Biege-Test oder der curl-retention - Test angewendet.

Gemäß obiger Formeln und allen folgenden Formeln steht eine chemische Bindung, die mit dem Symbol * gekennzeichnet ist, für eine freie Valenz des entsprechenden Strukturfragments.

Besonders vorteilhaft erweisen sich die Eigenschaften des erfindungsgemäßen Mittels, wenn es als Aerosolspray, Aerosolschaum, Pumpspray oder Pumpschaum konfektioniert wird. Diese bevorzugte Form der Konfektionierung wird später im Detail beschrieben.

Stärke ist ein Reservekohlenhydrat, das von vielen Pflanzen in Form von üblicherweise 1 bis 200 $\mu$m großen Stärkekörnern (Granula) in verschiedenen Pflanzenteilen gespeichert wird, z.B. in Knollen oder Wurzeln, Getreidesamen, Früchten sowie im Mark. Eine erfindungsgemäß verwendbare nichtionische, mittels Propylenoxid modifizierte Stärke kann sich aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok ableiten. Besonders ausgeprägte erfindungsgemäße Effekte werden durch nichtionische, mittels Propylenoxid modifizierte Tapioka Stärke bzw. nichtionische, mittels Propylenoxid modifizierte Kartoffelstärke bzw. durch Gemische beider vorgenannter Stärken, erzielt. Ganz besonders bevorzugt enthält das erfindungsgemäße Mittel mindestens eine nichtionische, mittels Propylenoxid modifizierte Kartoffelstärke.

Stärke gehört zu der Familie der Homoglycane und ist ein Polykondensationsprodukt von D-Glucose. Dabei besteht Stärke aus drei strukturell verschiedenen Polymeren der d-Glucopyranose, nämlich der Amylose, dem Amylopektin und einer sogenannten Zwischenfraktion. Höhere Pflanzen enthalten 0 bis 45 Gew.-% Amylose bezogen auf die Trockensubstanz.

Die Zwischenfraktion, die auch als anormales Amylopektin bezeichnet wird, steht strukturell zwischen der Amylose und dem Amylopektin. Die im Rahmen dieser Anmeldung definierten Mengenangaben für Amylopektin umfassen die Zwischenfraktion.

Es ist erfindungsgemäß bevorzugt, wenn die nichtionische, mittels Propylenoxid modifizierte Stärke einen

Amylosegehalt von kleiner 25 Gew.-%, insbesondere von kleiner 20 Gew.-%, -jeweils bezogen auf das Gewischt der modifizierten Stärke - besitzt. Es zeigte sich, dass sich zur Erreichung des erfindungsgemäßen Effektes besonders eine Stärke eignet, die 17 bis 22 Gew.-% Amylose und 78 bis 83 Gew.-% Amylopektin enthält.

Amylose besteht aus überwiegend linear α-1,4-glycosidisch verknüpfter d-Glucose, Mr 50000-150000. Die resultierenden Ketten bilden in der Stärke Doppelhelices.

Amylopektin enthält neben den für Amylose beschriebenen α-1,4-Verknüpfungen auch in einer Menge von 4 bis 6% α-1,6-Bindungen als Verzweigungsstellen. Der durchschnittliche Abstand zwischen den Verzweigungsstellen beträgt etwa 12 bis 17 Glucose-Einheiten. Die Molmasse von $10^7$ bis 7 · 108 entspricht ca. $10^5$ Glucose-Einheiten, womit Amylopektin zu den größten Biopolymeren gehört. Besagte Verzweigungen sind derart über das Molekül verteilt, dass sich eine Büschelstruktur mit relativ kurzen Seitenketten entwickelt. Zwei dieser Seitenketten bilden jeweils eine Doppelhelix. Durch die vielen Verzweigungsstellen ist Amylopektin in Wasser relativ gut löslich.

Unter einer nichtionischen, mittels Propylenoxid modifizierten Stärke wird erfindungsgemäß ein Reaktionsprodukt aus einer Stärke und Propylenoxid verstanden. Ein solches Reaktionsprodukt umfasst mindestens eine Struktureinheit der Formel (PS),

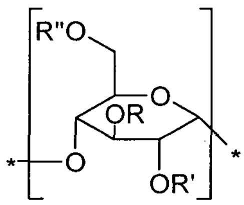

(PS),

worin mindestens ein Rest R, R' oder R" für eine Gruppe der Formel

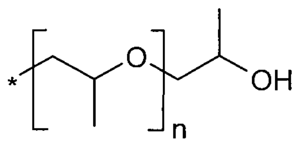

mit n ≥0 steht

und maximal 2 der Reste aus R, R', R" für ein Wasserstoffatom steht. Die nichtionischen, mittels Propylenoxid modifizierten Stärken werden beispielsweise durch Umsetzung einer nativen Stärke mit Propylenoxid bereitgestellt. Vor der Modifikation mit Propylenoxid kann die Stärke diversen physikalischen oder chemischen Prozessen ausgesetzt worden sein, wie z.B. einer Wärmebehandlung, Scherung, einer thermischen, säurehydrolytischen, oxidativen oder enzymatischen Spaltung, etc.

Es ist erfindungsgemäß bevorzugt, wenn die nichtionische, mittels Propylenoxid modifizierte Stärke in dem erfindungsgemäßen Mittel nicht in Form einzelner Stärkekörnchen (Granula) vorliegt. Zu diesem Zweck werden die Stärkekörner aufgeschlossen z.B. durch Hitze oder Scherung und die entsprechenden Polysaccharidmoleküle aus dem Verbund freigesetzt. Die freigesetzten Polysaccharidmoleküle werden nach oder vor der Freisetzung mittels Propylenoxid modifiziert.

Im Rahmen einer bevorzugten Ausführungsform ist die nichtionische, mittels Propylenoxid modifizierte Stärke verkleistert. Wird eine wässrige Suspension von Stärke erhitzt oder komprimiert, so beobachtet man bei einer kritischen Temperatur bzw. Druck eine tangentiale Quellung der Körper mit Verlust der Doppelbrechung, Änderung der Röntgenstruktur und abrupter Steigerung der Viskosität der Lösung. Diese Erscheinung wird Verkleisterung genannt.

Die erfindungsgemäßen nichtionischen, mittels Propylenoxid modifizierten Stärken liegen in dem erfindungsgemäßen Mittel in einer Molekulargewichtsverteilung vor. Erfindungsgemäß verwendete nichtionische, mittels Propylenoxid modifizierte Stärken weisen ein mittleres Molekulargewicht von 700 bis 1000 kDa (Gewichtsmittel) auf. Die Molekulargewichtsverteilung wurde experimentell mittels Gelfiltrationschromatographie gegen Dextran bestimmt. Das besagte Gewichtsmittel ist ein mittleres Molekulargewicht, welches das Totalgewicht der Moleküle verschiedenen Molekulargewichts berücksichtigt und nicht lediglich nur die Anzahl der Moleküle. Zur statistischen Berechnung des Gewichtsmittels wird zunächst der "Gewichtsbruch"

$$w_i = (N_i M_i) / [\Sigma(N_i M_i)]$$

definiert. Dieser gibt den Gewichtsanteil an Makromolekülen in der Probe an, die aus i Segmenten (z. B. Monomerbausteinen) der Masse $M_i$ bestehen und in der Probe $N_i$-mal vorkommen. Für das Gewichtsmittel des Molekulargewichts $M_w = \Sigma w_i M_i$ gilt damit

$$M_w = [\Sigma(N_i M^2_i)] / [\Sigma(N_i M_i)].$$

Zur Einstellung des Molekulargewichts wird die Stärke vor oder nach der Modifizierung mittels Propylenoxid einer mechanischen und/oder einer chemischen Behandlung unterzogen. Zur Molekulargewichtserhöhung kann die besagte Stärke vernetzt werden. Eine Vernetzung der nichtionischen, mittels Propylenoxid modifizierten Stärke liegt vor, wenn die linearen bzw. verzweigten Polysaccharid-Makromoleküle der Stärke kovalent durch ein Vernetzungsmittel unter Bildung eines dreidimensionalen, unlöslichen und nur noch quellbaren polymeren Netzwerkes verknüpft werden. Native Stärke gilt im Allgemeinen als unvernetzt und bedarf - falls eine Vernetzung gewünscht wäre - einer künstlichen Vernetzung mittels Synthesechemie. Eine solche künstliche Vernetzung lässt sich mit Vernetzungsmitteln durchführen. (Nichtionische, mittels Propylenoxid modifizierte) Stärken, die eine solche Vernetzung nicht aufweisen, sind unvernetzt.

Eine Vernetzung erfolgt beispielsweise durch das Vernetzungsmittel Epichlorhydrin. Hierzu wird eine 42 Gew.-%-ige Mischung von nichtionischer, mittels Propylenoxid modifizierter Stärke in Wasser hergestellt, in diese die gewünschte Menge Epichlorhydrin bei Raumtemperatur eingerührt und nach einer Rührzeit von 1 bis 5 Stunden unter Kontrolle der Viskosität nach erreichen der Zielviskosität die vernetzte Stärke nach gängigen Verfahren isoliert.

Es ist jedoch erfindungsgemäß besonders bevorzugt, wenn die erfindungsgemäß verwendeten Mittel mindestens eine unvernetzte, nichtionische, mittels Propylenoxid modifizierte Stärke enthalten.

Der Gehalt an Propylenoxid der Stärke wirkt sich auf die Feinabstimmung des Frisurenhalts mit der Frisurenflexibilität und der Stabilität der kosmetischen Mittel aus. Erfindungsgemäß verwendete nichtionische, mittels Propylenoxid modifizierte Stärke weist - bezogen auf das Gewicht der modifizierten Stärke - einen Propylenoxidgehalt von 1 bis 20 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%, auf. Der Propylenoxidgehalt lässt sich beispielsweise nach Durchführung einer Hodges-Spaltung mit der Methode gemäß DIN EN 13268 bestimmen.

Ferner hat es sich erwiesen, dass sich solche kosmetischen Mittel zur erfindungsgemäßen Verwendung hervorragend eignen, in denen die nichtionische, mittels Propylenoxid modifizierte Stärke in einer 43 Gew.-%-igen wässrigen Lösung eine bevorzugte Viskosität im Bereich von 150 bis 1500000 mPa·s (Brookfield Viskosimeter, Spindel #7 bei 20°C und 20 U/min) aufweist. Besonders geeignete mittels Propylenoxid modifizierte Stärken weisen Viskositäten von 10000 bis 200000 mPa·s, besonders bevorzugt von 25000 bis 180000 mPa·s, (jeweils gemessen unter den zuvor genannten Bedingungen) auf.

Eine erfindungsgemäße besonders bevorzugte nichtionische, mittels Propylenoxid modifizierte Stärke ist unvernetzt, weist ein mittleres Molekulargewicht (Gewichtsmittel) von 700 bis 1000 kDa, auf und hat - bezogen auf das Gewicht der modifizierten Stärke - einen Propylenoxidgehalt von 1 bis 20 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%. Dabei handelt es sich wiederum bevorzugt um Tapiokastärke oder Kartoffelstärke, insbesondere um Kartoffelstärke.

Eine erfindungsgemäß besonders bevorzugt verwendete nichtionische, mittels Propylenoxid modifizierte Stärke ist unvernetzt, weist ein mittleres Molekulargewicht (Gewichtsmittel) von 700 bis 1000 kDa, auf und hat - bezogen auf das Gewicht der modifizierten Stärke - einen Propylenoxidgehalt von 1 bis 20 Gew.-%, besonders bevorzugt einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%. Dabei handelt es sich wiederum bevorzugt um Tapiokastärke oder Kartoffelstärke, insbesondere um Kartoffelstärke.

Eine erfindungsgemäß ganz besonders bevorzugt verwendete nichtionische, mittels Propylenoxid modifizierte Kartoffelstärke ist unvernetzt, weist ein mittleres Molekulargewicht (Gewichtsmittel) von 700 bis 1000 kDa, auf und hat - bezogen auf das Gewicht der modifizierten Kartoffelstärke - einen Propylenoxidgehalt von 4 bis 12 Gew.-%, ganz besonders bevorzugt einen Propylenoxidgehalt von 9,5 bis 10,5 Gew.-% oder von 4,0 bis 6,0 Gew.-%.

Es ist erfindungsgemäß bevorzugt, wenn das erfindungsgemäß verwendete kosmetisches Mittel die nichtionische, mittels Propylenoxid modifizierte Stärke in einer Menge von 4,0 Gew.-% bis 15 Gew.-%, ganz besonders bevorzugt von 8,0 bis 12 Gew.-%, jeweils bezogen auf das Gewicht des Mittels, enthält.

Zusätzlich enthält das erfindungsgemäß verwendete Mittel zwingend mindestens ein filmbildendes und/oder festigendes Polymer.

Besonders bevorzugte filmbildende und/oder festigende Polymere sind anionisch. Unter einem anionischen Polymer wird erfindungsgemäß ein Polymer verstanden, das in einem protischen Lösemittel bei Standardbedingungen Struktureinheiten mit anionischen Gruppen, welche durch Gegenionen unter Erhaltung der Elektroneutralität kompensiert

werden müssen, trägt und keine Struktureinheiten mit permanent kationischen Gruppen aufweist. Unter anionische Gruppen fallen Carboxyl- und Sulfonsäuregruppen.

Erfindungsgemäß werden filmbildende und/oder festigende Polymere verwendet, die mindestens eine Struktureinheit der Formel (I) und mindestens eine Struktureinheit der Formel (II) umfassen,

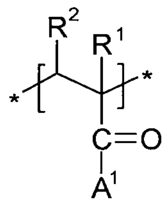 (I) 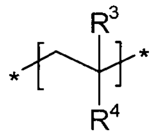 (II)

worin

$R^1$ und $R^2$ unabhängig voneinander für ein Wasserstoffatom oder eine Methylgruppe stehen, mit der Maßgabe, dass $R^1$ und $R^2$ nicht gleichzeitig für eine Methylgruppe stehen,

$R^3$ für ein Wasserstoffatom oder eine Methylgruppe steht,

$R^4$ für eine Carbamoyl-Gruppe, eine lineare oder verzweigte ($C_4$ bis $C_{12}$)-Alkylaminocarbonylgruppe, eine lineare oder verzweigte ($C_4$ bis $C_{12}$)-Alkylaminoethyl-aminocarbonylgruppe, eine lineare oder verzweigte ($C_4$ bis $C_{12}$)-Alkylaminopropyl-aminocarbonylgruppe, eine lineare oder verzweigte ($C_4$ bis $C_{12}$)-Alkyloxycarbonylgruppe, eine lineare oder verzweigte ($C_4$ bis $C_{12}$)-Alkylaminoethyloxycarbonylgruppe, eine lineare oder verzweigte ($C_4$ bis $C_{12}$)-Alkylaminopropyloxycarbonylgruppe, eine lineare oder verzweigte ($C_2$ bis $C_{12}$)-Acyloxygruppe steht,

$A^1$ für eine Hydroxygruppe oder einen organischen Rest mit mindestens einer Sulfonsäuregruppe steht, der über ein Sauerstoffatom oder eine Gruppe NH an das Strukturfragment bindet.

Die filmbildenden und/oder festigenden, vorzugsweise anionischen, filmbildenden und/oder anionischen, festigenden Polymere (b) sind in dem erfindungsgemäß verwendeten Mittel in einer Menge von 1,0 Gew.-% bis 9,0 Gew.-%, ganz besonders bevorzugt von 3,0 Gew.-% bis 8,0 Gew.-%, jeweils bezogen auf das Gewicht des erfindungsgemäß verwendeten Mittels, enthalten.

Es ist erfindungsgemäß bevorzugt, wenn das filmbildende anionische und/oder festigende anionische Polymer (b) mindestens eine Struktureinheit der Formel (I) enthält, die ausgewählt wird aus mindestens einer Struktureinheit der Formeln (I-1) bis (I-5)

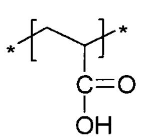 (I-1) 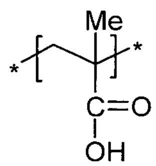 (I-2) 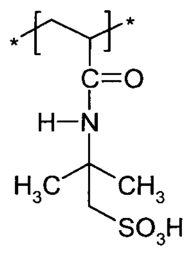 (I-3)

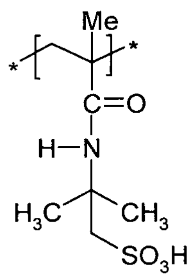 (I-4) 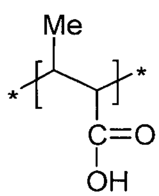 (I-5)

Dabei ist es wiederum besonders bevorzugt, wenn das Polymer (b) neben den obigen Struktureinheiten der Formeln (I) und (II) zusätzlich mindestens eine Struktureinheit der Formel (III) enthält

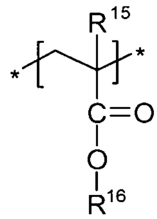

(III)

worin

$R^{15}$ steht für ein Wasserstoffatom oder eine Methylgruppe
$R^{16}$ steht für eine ($C_1$ bis $C_4$)-Alkylgruppe (insbesondere eine Methylgruppe oder eine Ethylgruppe).

Copolymere aus Methacrylsäure und Ethylacrylat und tert-Butylacrylat eignen sich beispielsweise als bevorzugt.

Es ist erfindungsgemäß besonders bevorzugt, wenn das filmbildende anionische und/oder festigende anionische Polymer (b) mindestens eine Struktureinheit der Formel (II) enthält, die ausgewählt wird aus mindestens einer Struktureinheit der Formeln (II-1) bis (II-15)

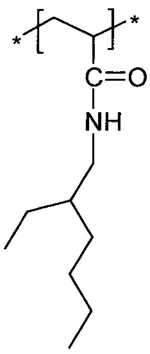

(II-1)

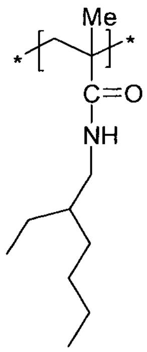

(II-2)

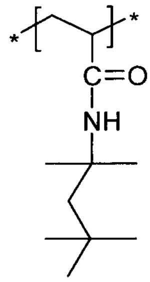

(II-3)

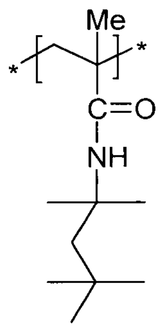

(II-4)

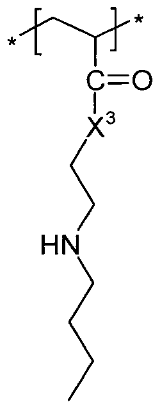

(II-5)

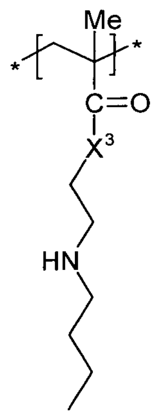

(II-6)

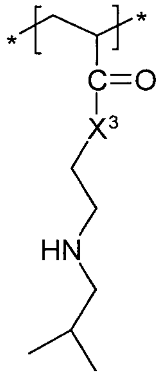 (II-7) 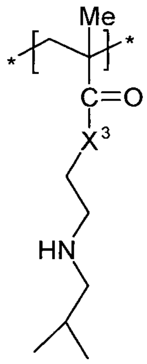 (II-8) 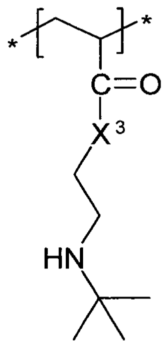 (II-9)

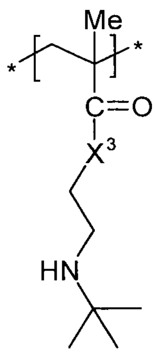 (II-10) 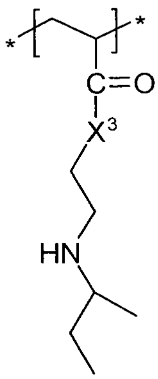 (II-11) 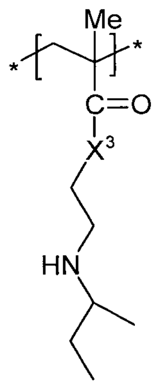 (II-12)

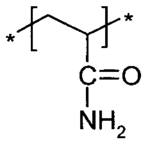 (II-13) 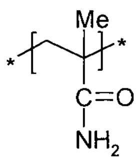 (II-14) 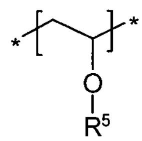 (II-15)

worin

$X^3$ für ein Sauerstoffatom oder eine Gruppe NH steht,
$R^5$ für eine ($C_2$ bis $C_{12}$)-Acylgruppe (insbesondere für Acetyl oder Neodecanoyl) steht.

Es ist erfindungsgemäß bevorzugt, wenn $X^3$ gemäß Formeln (II-5) bis (II-12) für ein Sauerstoffatom steht.

Im Rahmen einer ersten bevorzugten Ausführungsform der Erfindung enthält das erfindungsgemäß verwendete Mittel mindestens ein filmbildendes anionisches und/oder festigendes anionisches Polymer (b), welches mindestens eine Struktureinheit der Formel (I-1), mindestens eine Struktureinheit der Formel (II-3) und mindestens eine Struktureinheit der Formel (II-16) (insbesondere ausgewählt aus der Gruppe, die gebildet wird aus obigen Formeln (II-5) bis (II-12) mit der Maßgabe, dass $X^3$ für ein Sauerstoffatom steht),

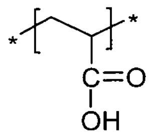 (I-1) 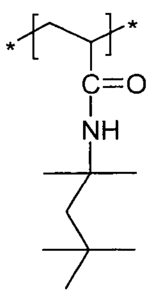 (II-3) 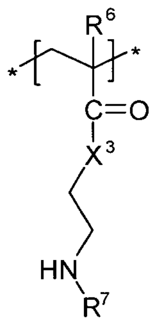 (II-16)

worin $X^3$ steht für ein Sauerstoffatom oder eine Gruppe NH,
$R^6$ steht für ein Wasserstoffatom oder eine Methylgruppe und
$R^7$ steht für eine Alkylgruppe mit 4 Kohlenstoffatomen (insbesondere n-Butyl, sec-Butyl, iso-Butyl oder tert-Butyl).

Dabei ist es wiederum besonders bevorzugt, wenn das Polymer (b) neben den obigen Struktureinheiten der Formeln (I-1), (II-3) und (II-16) zusätzlich mindestens eine Struktureinheit der Formel (III) enthält

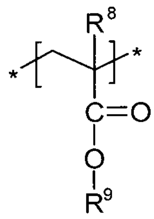
(III)

worin

$R^8$ steht für ein Wasserstoffatom oder eine Methylgruppe
$R^9$ steht für eine ($C_1$ bis $C_4$)-Alkylgruppe (insbesondere eine Methylgruppe oder eine Ethylgruppe).

Bevorzugte Polymere (b) dieser Art werden ausgewählt aus der Gruppe, die gebildet wird aus:

- Copolymeren aus Acrylsäure, ($C_1$ bis $C_4$)-Alkylacrylaten, $C_4$-Alkylaminoethylmethacrylat und $C_8$-Alkylacrylamid.

Ein Beispiel eines besonders bevorzugt im Rahmen dieser Ausführungsform verwendbaren Polymers (b) ist das unter dem Handelsnamen Amphomer® 028-4910 von der Firma National Starch erhältliche Polymer mit der INCI-Bezeichung Octylacrylamide/Acrylates/Butylaminoethylmethacrylate Copolymer.

Es gelten somit insbesondere solche Mittel im Rahmen dieser Ausführungsform als ganz besonders bevorzugt, die in einem kosmetisch akzeptablen Träger enthalten

(a) mindestens eine unvernetzte, nichtionische, mittels Propylenoxid modifizierte Stärke in einer Menge von 4,0 Gew.-% bis 15 Gew.-%, bezogen auf das Gewicht des Mittels, wobei die nichtionische, mittels Propylenoxid modifizierte Stärke ein mittleres Molekulargewicht (Gewichtsmittel) von 700 bis 1000 kDa aufweist und wobei die nichtionische, mittels Propylenoxid modifizierte Stärke einen Propylenoxidgehalt von 1 bis 20 Gew.-%, bezogen auf das Gewicht der modifizierten Stärke, aufweist,
und
(b) mindestens ein anionisches filmbildendes und/oder anionisches festigendes Polymer (b) in einer Menge von 1,0 Gew.-% bis 9,0 Gew.-%, bezogen auf das Gewicht des Mittels, welches mindestens eine Struktureinheit der Formel (I-1), mindestens eine Struktureinheit der Formel (II-3) und mindestens eine Struktureinheit der Formel (II-16) umfasst,

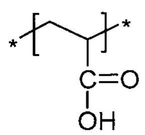
(I-1)
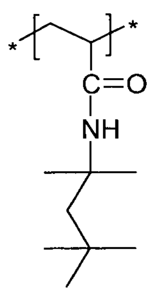
(II-3)
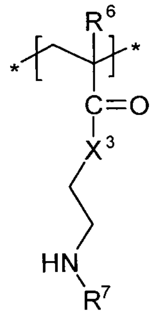
(II-16)

worin

$X^3$ steht für ein Sauerstoffatom oder eine Gruppe NH (insbesondere für ein Sauerstoffatom),
$R^6$ steht für ein Wasserstoffatom oder eine Methylgruppe (insbesondere für eine Methylgruppe) und

$R^7$ steht für eine Alkylgruppe mit 4 Kohlenstoffatomen (insbesondere n-Butyl, sec-Butyl, iso-Butyl oder tert-Butyl).

Dabei ist es wiederum besonders bevorzugt, wenn das Polymer (b) neben den obigen Struktureinheiten der Formeln (I-1), (II-3) und (II-16) zusätzlich mindestens eine Struktureinheit der Formel (III) enthält

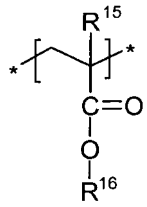

(III)

worin

$R^{15}$ steht für ein Wasserstoffatom oder eine Methylgruppe
$R^{16}$ steht für eine ($C_1$ bis $C_4$)-Alkylgruppe (insbesondere eine Methylgruppe oder eine Ethylgruppe).

Dabei ist es besonders bevorzugt, wenn für alle Ausführungsformen dieser ersten Ausführungsform, wenn die Struktureinheit der Formel (I-1) ganz oder teilweise neutralisiert vorliegt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Im Rahmen einer zweiten Ausführungsform gelten solche Mittel als erfindungsgemäß bevorzugt, die als anionisches filmbildendes und/oder anionisches festigendes Polymer (b) mindestens ein Polymer enthalten, das mindestens eine Struktureinheit der Formel (I-3) und mindestens eine Struktureinheit der Formel (I-13) enthalten

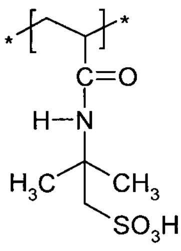

(I-3)

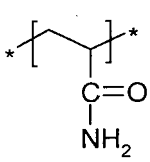

(II-13).

Bevorzugte Polymere (b) dieser Art werden ausgewählt aus mindestens einem Polymer der Gruppe, die gebildet wird aus

- Copolymeren aus 2-Acrylamido-2-methyl-propansulfonsäure und Acrylamid,
- Copolymeren aus 2-Acrylamido-2-methyl-propansulfonsäure, Acrylamid und Acrylsäure.

Polymere dieser Art werden beispielsweise in einer inversen Isohexadecan-Emulsion von der Firma Seppic unter dem Handelsnamen Sepigel® 305 (INCI-Bezeichnung: Polyacrylamide, C13-14 Isoparaffin, Laureth-7) oder Simulgel® 600 (INCI-Bezeichnung: Acrylamide/Acryloyldimethyltaurate Copolymer, Isohexadecane, Polysorbate-80) vertrieben.

Ein erfindungsgemäß besonders bevorzugt verwendetes Mittel ist dadurch gekennzeichnet, dass es als Polymer (b) ein Copolymer (b1) enthält.

Diese Copolymere (b1) lassen sich durch die allgemeine Formel

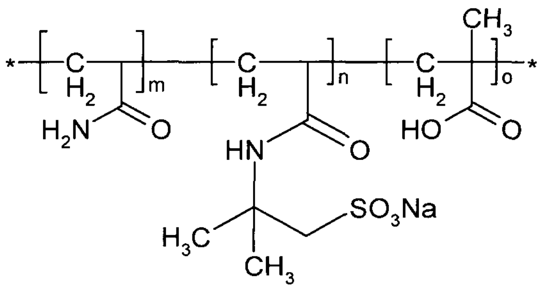

beschreiben, wobei die Indices m, n und o je nach Molmasse des Polymers variieren und nicht bedeuten sollen, dass es sich um Blockcopolymere handelt. Vielmehr können Struktureinheiten im Molekül statistisch verteilt vorliegen.

Besonders bevorzugt verwendete erfindungsgemäße Mittel sind dadurch gekennzeichnet, dass das Copolymer (b1) eine Molmasse von 50 bis 500 kDa, vorzugsweise von 100 bis 450 kDa, weiter bevorzugt von 150 bis 400 kDa und insbesondere von 200 bis 300 kDa aufweist.

Copolymere von Acrylamid mit Methacrylsäure und Acryloyldimethyltaurat sind beispielsweise unter dem Handelsnamen Acudyne® SCP (Rohm & Haas) erhältlich.

Es gelten daher weiterhin insbesondere solche Mittel im Rahmen dieser Ausführungsform als ganz besonders bevorzugt, die in einem kosmetisch akzeptablen Träger enthalten

(a) mindestens eine unvernetzte, nichtionische, mittels Propylenoxid modifizierte Stärke in einer Menge von 4,0 Gew.-% bis 15 Gew.-%, bezogen auf das Gewicht des Mittels, wobei die nichtionische, mittels Propylenoxid modifizierte Stärke ein mittleres Molekulargewicht (Gewichtsmittel) von 700 bis 1000 kDa aufweist und wobei die nichtionische, mittels Propylenoxid modifizierte Stärke einen Propylenoxidgehalt von 1 bis 20 Gew.-%, bezogen auf das Gewicht der modifizierten Stärke, aufweist,
und
(b) mindestens ein anionisches filmbildendes und/oder anionisches festigendes Polymer (b) in einer Menge von 1,0 Gew.-% bis 9,0 Gew.-%, bezogen auf das Gewicht des Mittels, das mindestens eine Struktureinheit der Formel (I-5) und mindestens eine Struktureinheit der Formel (I-13) umfasst

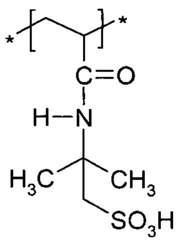
(I-3)

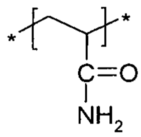
(II-13).

Im Rahmen einer dritten Ausführungsform gelten solche Mittel als erfindungsgemäß bevorzugt, die als anionisches filmbildendes und/oder anionisches festigendes Polymer (b) mindestens ein Polymer enthalten, das mindestens eine Struktureinheit der Formel (I-3) und mindestens eine Struktureinheit der Formel (I-13) enthalten

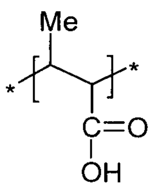
(I-5)

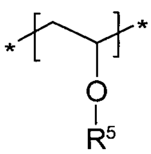
(II-15)

worin $R^5$ für eine ($C_2$ bis $C_{12}$)-Acylgruppe (insbesondere für Acetyl oder Neodecanoyl) steht. Besonders bevorzugte Polymere (b) dieser Art werden ausgewählt aus mindestens einem Polymer der Gruppe, die gebildet wird aus

- Copolymeren aus Vinylacetat und Crotonsäure.
- Copolymeren aus Vinylpropionat und Crotonsäure,

- Copolymeren aus Vinylneodecanoat, Vinylacetat und Crotonsäure.

Solche Copolymere werden beispielsweise von der Firma Clariant unter dem Handelsnamen Aristoflex A 60 (INCI-Bezeichnung: VA/Crotonates Copolymer) in einem Isopropanol-WasserGemisch (60 Gew.-% Aktivsubstanz), von der Firma BASF unter dem Handelsnamen Luviset CA 66 (Vinylacetat/Crotonsäure-Copolymer 90:10, INCI-Bezeichnung VA/Crotonates Copolymer) bereitgestellt, von der Firma National Starch unter dem Handelsnamen Resyn 28-2942 bzw. Resyn 28-2930 (INCI-Bezeichnung: VA/Crotonates/Vinyl Neodecanoate Copolymer) bereitgestellt.
Es gelten daher weiterhin insbesondere solche Mittel im Rahmen dieser Ausführungsform als ganz besonders bevorzugt, die in einem kosmetisch akzeptablen Träger enthalten

(a) mindestens eine unvernetzte, nichtionische, mittels Propylenoxid modifizierte Stärke in einer Menge von 4,0 Gew.-% bis 15 Gew.-%, bezogen auf das Gewicht des Mittels, wobei die nichtionische, mittels Propylenoxid modifizierte Stärke ein mittleres Molekulargewicht (Gewichtsmittel) von 700 bis 1000 kDa aufweist und wobei die nichtionische, mittels Propylenoxid modifizierte Stärke einen Propylenoxidgehalt von 1 bis 20 Gew.-%, bezogen auf das Gewicht der modifizierten Stärke, aufweist,
und
(b) mindestens ein anionisches filmbildendes und/oder anionisches festigendes Polymer (b) in einer Menge von 1,0 Gew.-% bis 9,0 Gew.-%, bezogen auf das Gewicht des Mittels, das mindestens eine Struktureinheit der Formel (I-5) und mindestens eine Struktureinheit der Formel (I-15) umfasst

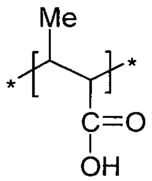
(I-5)
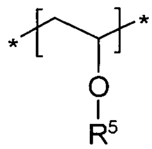
(II-15)

worin $R^5$ für eine ($C_2$ bis $C_{12}$)-Acylgruppe (insbesondere für Acetyl oder Neodecanoyl) steht.

Im Rahmen dieser Ausführungsformen eignen sich die zuvor genannten bevorzugten Ausführungsformen des amphiphilen, kationischen Polymers (a) als bevorzugt (*vide supra*). Gleichsam gelten alle zuvor genannten bevorzugten Mengenangaben bezüglich der Polymerkomponenten (a) und (b) zur Verwendung des erfindungsgemäßen Mittels auch für diese Ausführungsformen *mutatis mutandis* als bevorzugt.
Dabei ist es besonders bevorzugt, wenn für alle Ausführungsformen dieser dritten Ausführungsform, die Struktureinheit der Formel (I-5) ganz oder teilweise neutralisiert vorliegt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin),3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.
Zur Intensivierung des erfindungsgemäßen Effektes enthalten die erfindungsgemäßen Mittel vorzugsweise zusätzlich mindestens ein Tensid, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben.
Die zusätzlichen Tenside sind in dem erfindungsgemäß verwendeten Mittel bevorzugt in einer Menge von 0,01 Gew.-% bis 5 Gew.-%, besonders bevorzugt von 0,05 Gew.-% bis 0,5 Gew.-%, jeweils bezogen auf das Gewicht des Mittels, enthalten.
Es hat sich als besonders bevorzugt erwiesen, wenn die erfindungsgemäß verwendeten Mittel zusätzlich mindestens ein nichtionisches Tensid enthalten.
Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,

- mit einem Methyl- oder $C_2$ - $C_6$ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

$$R^1CO\text{-}(OCH_2CHR^2)_wOR^3 \qquad \text{(E4-I)}$$

in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,

- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

$$R^4O\text{-}[G]_p \qquad \text{(E4-II)}$$

in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside $C_{12}$-$C_{30}$-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Ganz besonders bevorzugt enthalten die erfindungsgemäßen Mittel als Tensid mindestens ein Anlagerungsprodukt von 15 bis 100 mol Ethylenoxid, insbesondere von 15 bis 50 mol Ethylenoxid an einen linearen oder verzweigten (insbesondere linearen) Fettalkohol mit 8 bis 22 Kohlenstoffatomen. Es handelt sich dabei ganz besonders bevorzugt um Ceteareth-15, Ceteareth-25 oder Ceteareth-50, welche als Eumulgin® CS 15 (COGNIS), Cremophor A25 (BASF SE) bzw. Eumulgin® CS 50 (COGNIS) vermarktet werden.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,

- Alpha-Sulfofettsäuremethylester von Fettsäuren mit 8 bis 30 C-Atomen,
- Alkylsulfate und Alkylpolyglykolethersulfate der Formel $R-O(CH_2-CH_2O)_x-OSO_3H$, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- sulfatierte Fettsäurealkylenglykolester der Formel (E1-II)

$$R^7CO(AlkO)_nSO_3M \qquad (E1\text{-}II)$$

  in der $R^7CO$- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Kation steht, wie sie in der DE-OS 197 36 906 beschrieben sind,
- Amidethercarbonsäuren,
- Kondensationsprodukte aus $C_8$ - $C_{30}$ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Als zwitterionische Tenside werden solche oberflächenaktiven Verbindungen bezeichnet, die im Molekül mindestens eine quartäre Ammoniumgruppe und mindestens eine $-COO^{(-)}$ - oder $-SO_3^{(-)}$ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer $C_8$ - $C_{24}$- Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das $C_{12}$ - $C_{18}$ - Acylsarcosin.

Die erfindungsgemäß verwendeten Mittel enthalten die Inhalts- bzw- Wirkstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Es ist erfindungsgemäß bevorzugt mindestens einen ($C_1$ bis $C_4$)-Monoalkylalkohol in den erfindungsgemäßen Mitteln insbesondere in einer Menge von 1 bis 50 Gew.-% insbesondere von 5 bis 30 Gew.-% einzusetzen. Dies ist wiederum insbesondere für die Konfektionierung als Pumpschaum oder Aerosolschaum bevorzugt.

Als zusätzliche Co-Solventien können organische Lösungsmittel oder ein Gemisch aus Lösungsmitteln mit

einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan.

Weitere, besonders bevorzugte wasserlösliche Lösungsmittel sind Glycerin, Ethylenglykol und Propylenglykol in einer Menge bis 30 Gew.-% bezogen auf das gesamte Mittel.

Insbesondere der Zusatz von Glycerin und/oder Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Glycerin und/oder Propylenglykol und/oder Polyethylenglykol und/oder Polypropylenglykol bezogen auf das gesamte Mittel.

Die Mittel weisen bevorzugt einen pH-Wert von 2 bis 11 auf. Besonders bevorzugt ist der pH-Bereich zwischen 4,0 bis 9,0, vorzugsweise zwischen 4,5 bis 7,5 und insbesondere zwischen 5,0 bis 6,0. Die Angaben zum pH-Wert beziehen sich dabei im Sinne dieser Schrift auf den pH-Wert bei 25°C, sofern nichts anderes vermerkt ist.

Die erfindungsgemäß verwendeten Mittel können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Siliciumorganischer Verbindungen. Zunächst werden hierunter die Dimethiconole verstanden.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone bilden die zweite Gruppe der Silikone, welche erfindungsgemäß enthalten sein können. Diese können sowohl linear als auch verzweigt als auch cyclisch oder cyclisch und verzweigt sein.

Dimethiconcopolyole (S3) bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning ® 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 $\mu$m, bevorzugt 0,01 bis 100 $\mu$m, besonders bevorzugt 0,01 bis 20 $\mu$m und ganz besonders bevorzugt 0,01 bis 10 $\mu$m. Die Teilchengröße wird dabei nach der

Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Besonders geeignete Silikone sind aminofunktionelle Silikone, insbesondere die Silikone, die unter der INCI-Bezeichnung Amodimethicone zusammengefasst sind. Daher ist es erfindungsgemäß bevorzugt, wenn die erfindungsgemäßen Mittel zusätzlich mindestens ein aminofunktionelles Silikon enthalten. Darunter sind Silikone zu verstehen, welche mindestens eine, gegebenenfalls substituierte, Aminogruppe aufweisen. Diese Silikone werden nach der INCI-Deklaration als Amodimethicone bezeichnet und sind beispielsweise in Form einer Emulsion als Handelsprodukt Dow Corning® 939 oder als Handelsprodukt Dow Corning® 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Die Mittel enthalten die Silikone bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex), Sericin (Pentapharm) und Kerasol® (Croda) vertrieben.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a. Vitamin $B_1$ (Thiamin), Vitamin $B_2$ (Riboflavin), Vitamin $B_3$ (Nicotinsäure und/oder Nicotinsäureamid (Niacinamid)), Vitamin $B_5$ (Pantothensäure, Panthenol und Pantolacton), Vitamin $B_6$ (Pyridoxin sowie Pyridoxamin und Pyridoxal), Vitamin C (Ascorbinsäure), Vitamin E (Tocopherole, insbesondere α-Tocopherol), Vitamin F (Linolsäure und/oder Linolensäure), Vitamin H.

Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt.

Ganz besonders bevorzugt wird als Pflegestoff D-Panthenol, gegebenenfalls in Kombination mit mindestens einem der oben genannten Silikonderivate eingesetzt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt

gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäß verwendeten Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Weiterhin kann es bevorzugt sein, in den erfindungsgemäß verwendeten Mitteln Mischungen aus mehreren, insbesondere aus zwei, verschiedenen Pflanzenextrakten einzusetzen.

Auch Mono- bzw. Oligosaccharide können als Pflegestoff in den erfindungsgemäßen Mitteln eingesetzt werden. Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist. Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Die Mono- bzw. Oligosaccharide sind in den erfindungsgemäß verwendeten Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC® sowie Phospholipid SV® vertrieben. Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).

- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I),

$$\begin{array}{l} CH_2O(CH_2CH_2O)_mR^1 \\ | \\ CHO(CH_2CH_2O)_nR^2 \\ | \\ CH_2O(CH_2CH_2O)_qR^3 \end{array} \qquad (D4\text{-}I)$$

in der $R^1$, $R^2$ und $R^3$ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht $R^1$ für einen Acylrest und $R^2$ und $R^3$ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäß verwendeten Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbare UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, $\alpha$-(2-Oxoborn-3-yliden)-toluol-4-sulfonsäure und deren Salze, ethoxylierte 4-Aminobenzoesäure-ethylester (PEG-25 PABA; Uvinul®P 25), 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz (Benzophenone-4; Uvinul®MS 40; Uvasorb®S 5), 3-(4'-Methylbenzyliden)-D,L-Campher, 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon, 1,1'-Diphenylacrylonitrilsäure-2-ethylhexyl-ester, o-Aminobenzoesäurementhylester, 2,2',4,4'-Tetrahydroxybenzophenon, 2,2'-Dihydroxy-4,4'-dimethoxybenzophenon, 2,2'-Dihydroxy-4,4'-dimethoxybenzophenon-5-natriumsulfonat und 2-Cyano-3,3-diphenylacrylsäure-2'-ethylhexylester. Bevorzugt sind 2-Hydroxy-4-methoxybenzophenon-5-sulfonsäure und deren Natriumsalz und/oder ethoxylierte 4-Aminobenzoesäure-ethylester.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäß verwendete Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Bevorzugt werden kationische direktziehende Farbstoffe eingesetzt. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die Farbstoffe, die auch unter den Bezeichnungen Basic Yellow 87, Basic Orange 31 und Basic Red 51 bekannt sind, sind ganz besonders bevorzugte kationische direktziehende Farbstoffe der Gruppe (c). Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäß verwendeten Mittel gemäß dieser Ausführungsform enthalten die direktziehenden Farbstoffe bevorzugt in einer Menge von 0,001 bis 20 Gew.-%, bezogen auf das gesamte Mittel.

Es ist erfindungsgemäß bevorzugt, dass die erfindungsgemäß verwendeten Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Formulierung der erfindungsgemäß verwendeten Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt.

Vorzugsweise werden die erfindungsgemäß verwendeten Mittel als Pumpspray, Aerosolspray, Pumpschaum oder Aerosolschaum.

Hierzu werden die erfindungsgemäßen Mittel in einer Abgabevorrichtung konfektioniert, die entweder ein zusätzlich mit einem Treibmittel befüllter Druckgasbehälter ("Aerosolbehälter") oder ein Nichtaerosolbehälter darstellt.

Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pumpsystem verteilt wird.

Insbesondere bevorzugt sind die erfindungsgemäß verwendeten Mittel als Aerosolhaarschaum oder Aerosolhaarspray konfektioniert. Das erfindungsgemäße Mittel enthält daher bevorzugt zusätzlich mindestens ein Treibmittel.

Erfindungsgemäß geeignete Treibmittel sind beispielsweise ausgewählt aus $N_2O$, Dimethylether, $CO_2$, Luft,

Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus.

Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus $N_2O$ Dimethylether, $CO_2$, Luft, Alkanen mit 3 oder 4 Kohlenstoffatomen (wie Propan, n-Butan, iso-Butan) sowie Mischungen daraus.

Eine bevorzugte Ausführungsform der erfindungsgemäß verwendeten Mittel sind Aerosolhaarschäume oder Aerosolhaarsprays, enthaltend das zuvor beschriebene erfindungsgemäße Mittel und mindestens ein Treibmittel.

Bevorzugte erfindungsgemäß verwendete Mittel und Treibmittel des Aerosolhaarschaums bzw. Aerosolhaarsprays, sowie die jeweiligen Mengen an Treibmittel entsprechen dem bereits oben Ausgeführten.

Die erfindungsgemäße Verwendung der zuvor beschriebenen Mittel ermöglicht eine Verbesserung des Farberhalts oxidativ gefärbter keratinischer Fasern.

Die nachfolgenden Beispiele sollen den Gegenstand der vorliegenden Erfindung erläutern ohne ihn in irgendeiner Weise zu beschränken.

Beispiele

Die folgenden Mengenangaben verstehen sich - soweit nichts anderes vermerkt ist - in Gewichtsprozent.

Folgende Rezepturen wurden durch Vermischen der angegebenen Rohstoffe bereitgestellt:

| Rohstoffe | A | B |
|---|---|---|
| PGE [1] | 10,0 | - |
| Polyvinylpyrrolidon (20%) | - | 22,5 |
| PVP/VA Copoylmer 60/40 | 7,0 | 7,0 |
| Gafquat 755N [2] | 5,2 | 5,2 |
| Dehyquart A [3] | 1,0 | 1,0 |
| Milchsäure (80%) | 0,08 | 0,08 |
| PEG-40 Hydrogenated Castor Oil | 0,4 | 0,4 |
| Wasser | add 100 | |

[1] nichtionische, mittels Propylenoxid modifizierte Kartoffelstärke (43 Gew.-% Aktivsubstanz in Wasser; Propylenoxidgehalt: 10 Gew.-%; Viskosität: 64000 mPas; Gewichtsmittel: 900 kDa)

[2] Copoylmer aus Vinylpyrrolidon und Dimethylaminoethylmethyacrylat (20 Gew.-% Aktivsubstanz in Wasser, INCI-Bezeichnung: Polyquaternium 11)

[3] Trimethylhexadecylammoniumchlorid (25 Gew.-% Aktivsubstanz in Wasser; INCI-Bezeichnung: Cetrimonium chloride)

Die Rezeptur A bewirkte nach Auftrag auf oxidativ gefärbte keratinische Fasern einen im Vergleich zur Rezeptur B verbesserten Farberhalt.

## Patentansprüche

1. Verwendung eines Mittels, enthaltend in einem kosmetisch akzeptablen Träger

   (a) mindestens eine nichtionische, mittels Propylenoxid modifizierte Stärke in einer Menge von 4,0 Gew.-% bis 15 Gew.-%, bezogen auf das Gewicht des Mittels, wobei die nichtionische, mittels Propylenoxid modifizierte Stärke ein mittleres Molekulargewicht (Gewichtsmittel) von 700 bis 1000 kDa aufweist und wobei die nichtionische, mittels Propylenoxid modifizierte Stärke einen Propylenoxidgehalt von 1 bis 20 Gew.-%, bezogen auf das Gewicht der modifizierten Stärke, aufweist,
   und
   (b) mindestens ein filmbildendes und/oder festigendes Polymer in einer Menge von 1,0 Gew.-% bis 9,0 Gew.-%, bezogen auf das Gewicht des Mittels, wobei das filmbildende und/oder festigende Polymer mindestens eine Struktureinheit der Formel (I) und mindestens eine Struktureinheit der Formel (II) umfasst,

   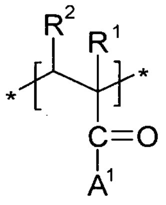 (I) 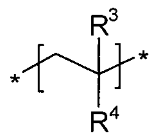 (II)

   worin

   $R^1$ und $R^2$ unabhängig voneinander für ein Wasserstoffatom oder eine Methylgruppe stehen, mit der Maßgabe, dass $R^1$ und $R^2$ nicht gleichzeitig für eine Methylgruppe stehen,
   $R^3$ für ein Wasserstoffatom oder eine Methylgruppe steht,
   $R^4$ für eine Carbamoyl-Gruppe, eine lineare oder verzweigte ($C_4$ bis $C_{12}$)-Alkylaminocarbonylgruppe, eine lineare oder verzweigte ($C_4$ bis $C_{12}$)-Alkylaminoethylaminocarbonylgruppe, eine lineare oder verzweigte ($C_4$ bis $C_{12}$)-Alkylaminopropylaminocarbonylgruppe, eine lineare oder verzweigte ($C_4$ bis $C_{12}$)-Alkyloxycarbonylgruppe, eine lineare oder verzweigte ($C_4$ bis $C_{12}$)-Alkylaminoethyloxycarbonylgruppe, eine lineare oder verzweigte ($C_4$ bis $C_{12}$)-Alkylaminopropyloxycarbonylgruppe, eine lineare oder verzweigte ($C_2$ bis $C_{12}$)-Acyloxygruppe steht,
   $A^1$ für eine Hydroxygruppe oder einen organischen Rest mit mindestens einer Sulfonsäuregruppe steht, der über ein Sauerstoffatom oder eine Gruppe NH an das Strukturfragment bindet.

   zur Verbesserung des Farberhalts oxidativ gefärbter keratinhaltiger Fasern, insbesondere oxidativ gefärbten menschlichen Haars.

2. Verwendung nach Anspruch 1, **dadurch gekennzeichnet, dass** die nichtionische, mittels Propylenoxid modifizierte Stärke eine nichtionische, mittels Propylenoxid modifizierte Tapioka Stärke oder eine nichtionische, mittels Propylenoxid modifizierte Kartoffelstärke oder ein Gemisch beider vorgenannter Stärken ist.

3. Verwendung nach einem der vorherigen Ansprüche, **dadurch gekennzeichnet, dass** das Mittel die nichtionische, mittels Propylenoxid modifizierte Stärke in einer Menge von 8,0 bis 12 Gew.-%, bezogen auf das Gewicht des Mittels, enthält.

4. Verwendung nach einem der vorherigen Ansprüche, **dadurch gekennzeichnet, dass** die nichtionische, mittels Propylenoxid modifizierte Stärke einen Propylenoxidgehalt von 4 bis 12 Gew.-%, besonders bevorzugt einen Propylenoxidgehalt von 9,5 bis 10,5 Gew.-% oder von 4,0 bis 6,0 Gew.-%, -jeweils bezogen auf das Gewicht der modifizierten Stärke - aufweist.

5. Verwendung nach einem der vorherigen Ansprüche, **dadurch gekennzeichnet, dass** als nichtionische, mittels Propylenoxid modifizierte Stärke mindestens eine unvernetzte, nichtionische, mittels Propylenoxid modifizierte Stärke enthalten ist.

6. Verwendung nach einem der vorherigen Ansprüche, **dadurch gekennzeichnet, dass** das filmbildende anionische und/oder festigende anionische Polymer (b) mindestens eine Struktureinheit der Formel (I) enthält, die ausgewählt wird aus mindestens einer Struktureinheit der Formeln (I-1) bis (I-5)

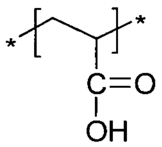
(I-1)
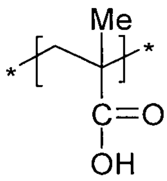
(I-2)
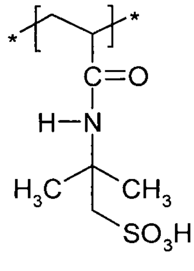
(I-3)

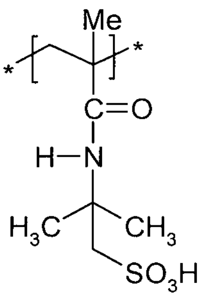
(I-4)
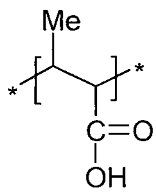
(I-5).

7. Verwendung nach einem der vorherigen Ansprüche, **dadurch gekennzeichnet, dass** das filmbildende anionische und/oder festigende anionische Polymer (b) mindestens eine Struktureinheit der Formel (II) enthält, die ausgewählt wird aus mindestens einer Struktureinheit der Formeln (II-1) bis (II-15)

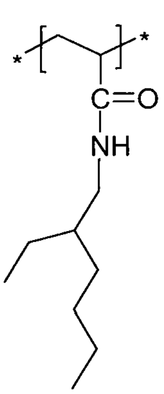
(II-1)
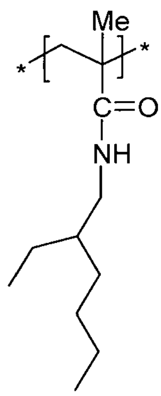
(II-2)
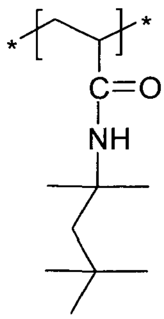
(II-3)

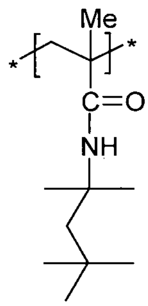
(II-4)

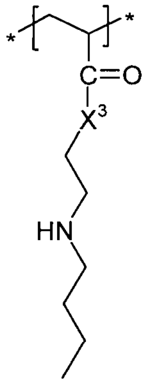
(II-5)

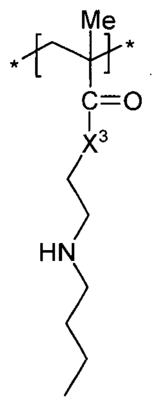
(II-6)

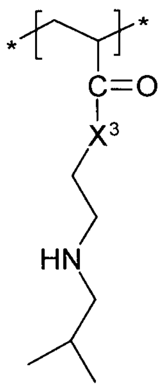
(II-7)

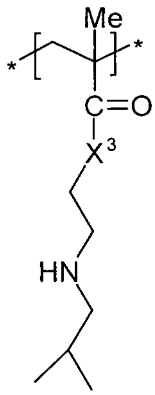
(II-8)

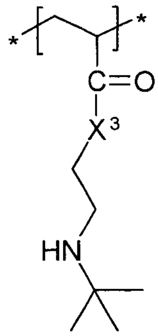
(II-9)

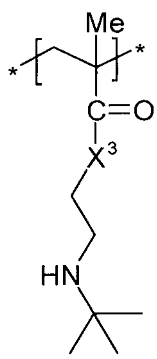
(II-10)

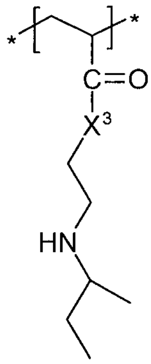
(II-11)

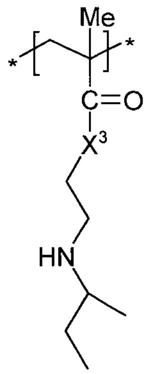
(II-12)

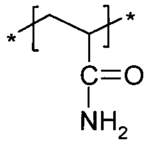
(II-13)

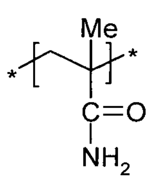
(II-14)

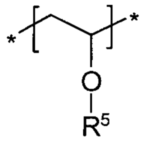
(II-15)

worin

$X^3$ für ein Sauerstoffatom oder eine Gruppe NH steht,
$R^5$ für eine ($C_2$ bis $C_{12}$)-Acylgruppe (insbesondere für Acetyl oder Neodecanoyl) steht.

**8.** Verwendung nach einem der vorherigen Ansprüche, **dadurch gekennzeichnet, dass** das filmbildende anionische und/oder festigende anionische Polymer (b) ausgewählt wird aus mindestens einem Polymer der Gruppe, die gebildet

wird aus

- Copolymeren aus Acrylsäure, ($C_1$ bis $C_4$)-Alkylacrylaten, $C_4$-Alkylaminoethylmethacrylat und $C_8$-Alkylacrylamid,
- Copolymeren aus 2-Acrylamido-2-methyl-propansulfonsäure und Acrylamid,
- Copolymeren aus 2-Acrylamido-2-methyl-propansulfonsäure, Acrylamid und Acrylsäure,
- Copolymeren aus Vinylacetat und Crotonsäure.
- Copolymeren aus Vinylpropionat und Crotonsäure,
- Copolymeren aus Vinylneodecanoat, Vinylacetat und Crotonsäure,
- Copolymeren aus Methacrylsäure und Ethylacrylat und tert-Butylacrylat.

**9.** Verwendung nach einem der vorherigen Ansprüche, **dadurch gekennzeichnet, dass** das Mittel die anionischen filmbildenden und/oder anionischen festigenden Polymere (b) in einer Menge von 3,0 Gew.-% bis 8,0 Gew.-%, jeweils bezogen auf das Gewicht des Mittels, enthält.

**10.** Verwendung nach einem der vorherigen Ansprüche, **dadurch gekennzeichnet, dass** das Mittel in Form eines Aerosolschaums oder Aerosolsprays vorliegt.

**11.** Verwendung nach einem der vorherigen Ansprüche, **dadurch gekennzeichnet, dass** das Mittel eine pH-Wert (25°C) von 4,0 bis 9,0, vorzugsweise von 4,5 bis 7,5 und insbesondere von 5,0 bis 6,0 auf weist.

**12.** Verwendung nach einem der vorherigen Ansprüche zur Verbesserung der Waschechtheit oxidativ gefärbter keratinhaltiger Fasern, insbesondere oxidativ gefärbten menschlichen Haars.

## Claims

**1.** The use of an agent containing, in a cosmetically acceptable carrier,

a) at least one non-ionic, propylene oxide-modified starch in an amount of from 4.0 wt.% to 15 wt.% based on the weight of the agent, wherein the non-ionic, propylene oxide-modified starch has an average molecular weight (weight average) of from 700 to 1000 kDa, and wherein the non-ionic, propylene oxide-modified starch has a propylene oxide content of from 1 to 20 wt.% based on the weight of the modified starch,
and
b) at least one film-forming and/or setting polymer in an amount of from 1.0 wt.% to 9.0 wt.%, based on the weight of the agent, wherein the film-forming and/or setting polymer comprises at least one structural unit of formula (I) and at least one structural unit of formula (II),

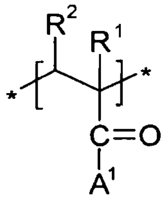 (I)

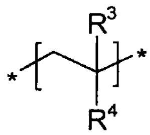 (II)

in which

$R^1$ und $R^2$ denote, independently of one another, a hydrogen atom or a methyl group, provided that $R^1$ and $R^2$ do not simultaneously denote a methyl group,
$R^3$ denotes a hydrogen atom or a methyl group,
$R^4$ denotes a carbamoyl group, a linear or branched ($C_4$ to $C_{12}$) alkyl aminocarbonyl group, a linear or branched ($C_4$ to $C_{12}$) alkyl aminoethyl aminocarbonyl group, a linear or branched ($C_4$ to $C_{12}$) alkyl aminopropyl aminocarbonyl group, a linear or branched ($C_4$ to $C_{12}$) alkyl oxycarbonyl group, a linear or branched ($C_4$ to $C_{12}$) alkyl aminoethyl oxycarbonyl group, a linear or branched ($C_2$ to $C_{12}$) alkyl aminopropyl oxycarbonyl group, a linear or branched ($C_2$ to $C_{12}$) acyloxy group,
$A^1$ denotes a hydroxy group or an organic functional group that has at least one sulfonic acid group and binds to the structural fragment via an oxygen atom or an NH group,

for improving the color retention of oxidatively colored keratin-containing fibers, in particular oxidatively colored human hair.

2. The use according to claim 1, **characterized in that** the non-ionic, propylene oxide-modified starch is a non-ionic, propylene oxide-modified tapioca starch or a non-ionic, propylene oxide-modified potato starch or a mixture of the two aforementioned starches.

3. The use according to either of the preceding claims, **characterized in that** the agent contains the non-ionic, propylene oxide-modified starch in an amount of from 8.0 to 12 wt.% based on the weight of the agent.

4. The use according to one of the preceding claims, **characterized in that** the non-Ionic, propylene oxide-modified starch has a propylene oxide content of from 4 to 12 wt.%, particularly preferably a propylene oxide content of from 9.5 to 10.5 wt.% or from 4.0 to 6.0 wt.%, based in each case on the weight of the modified starch.

5. The use according to one of the preceding claims, **characterized in that** at least one non-crosslinked, non-ionic, propylene oxide-modified starch is contained as a non-ionic, propylene oxide-modified starch.

6. The use according to one of the preceding claims, **characterized in that** the film-forming anionic and/or setting anionic polymer (b) contains at least one structural unit of formula (I), which is selected from at least one structural unit of formulas (I-1) to (I-5)

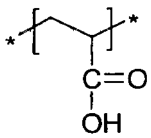

(I-1)

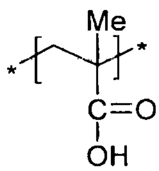

(I-2)

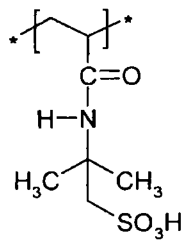

(I-3)

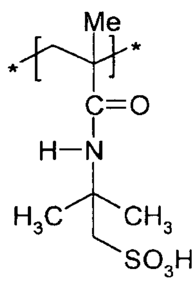

(I-4)

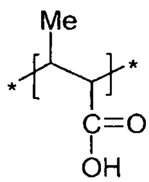

(I-5).

7. The use according to one of the preceding claims, **characterized in that** the film-forming anionic and/or setting anionic polymer (b) contains at least one structural unit of formula (II), which is selected from at least one structural unit of formulas (II-1) to (II-5),

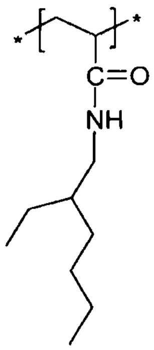
(II-1)

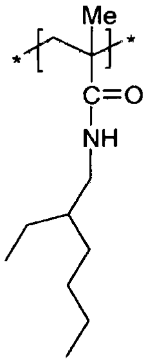
(II-2)

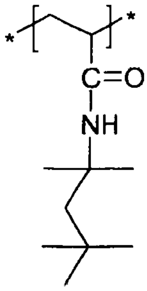
(II-3)

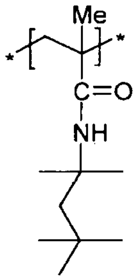
(II-4)

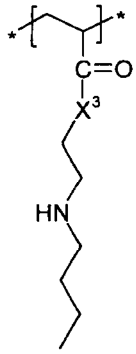
(II-5)

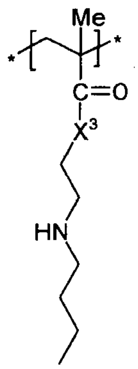
(II-6)

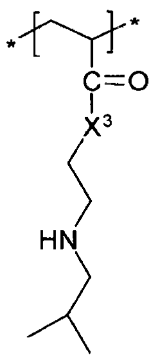
(II-7)

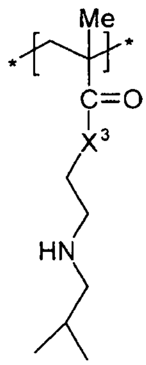
(II-8)

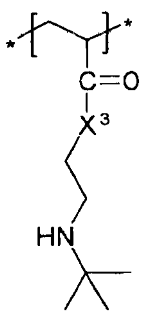
(II-9)

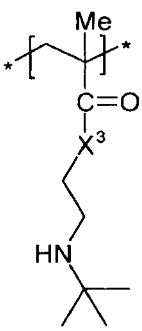
(II-10)

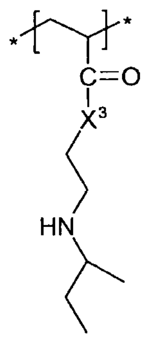
(II-11)

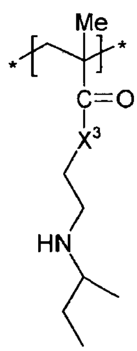
(II-12)

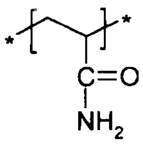 (II-13) 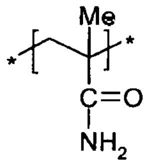 (II-14) 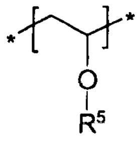 (II-15)

in which

$X^3$ denotes an oxygen atom or an NH group,
$R^5$ denotes a ($C_2$ to $C_{12}$) acyl group (in particular acetyl or neodecanoyl).

**8.** The use according to one of the preceding claims, **characterized in that** the film-forming anionic and/or setting anionic polymer (b) is selected from at least one polymer of the group formed by

- copolymers of acrylic acid, ($C_1$ to $C_4$) alkyl acrylates, $C_4$ alkyl aminoethyl methacrylate and $C_8$ alkyl acrylamide,
- copolymers of 2-acrylamido-2-methylpropane sulfonic acid and acrylamide,
- copolymers of 2-acrylamido-2-methylpropane sulfonic acid, acrylamide and acrylic acid,
- copolymers of vinyl acetate and crotonic acid,
- copolymers of vinyl propionate and crotonic acid,
- copolymers of vinyl neodecanoate, vinyl acetate and crotonic acid,
- copolymers of methacrylic acid and ethyl acrylate and tert-butyl acrylate.

**9.** The use according to one of the preceding claims, **characterized in that** the agent contains the anionic film-forming and/or anionic setting polymers (b) in an amount of from 3.0 wt.% to 8.0 wt.%, based in each case on the weight of the agent.

**10.** The use according to one of the preceding claims, **characterized in that** the agent is in the form of an aerosol mousse or an aerosol spray.

**11.** The use according to one of the preceding claims, **characterized in that** the agent has a pH (25°C) of from 4.0 to 9.0, preferably from 4.5 to 7.5 and in particular from 5.0 to 6.0.

**12.** The use according to one of the preceding claims for improving the washfastness of oxidatively colored keratin-containing fibers, in particular oxidatively colored human hair.

## Revendications

**1.** Utilisation d'un agent contenant dans un support cosmétiquement acceptable

(a) au moins un amidon non ionique, modifié par de l'oxyde de propylène, dans une quantité de 4,0% en poids à 15% en poids sur la base du poids de l'agent, l'amidon non ionique modifié par l'oxyde de propylène ayant un poids moléculaire moyen (moyenne en poids) de 700 à 1000 kDa, et l'amidon non ionique modifié par l'oxyde de propylène ayant une teneur en oxyde de propylène de 1 à 20% en poids par rapport au poids de l'amidon modifié,
et
(b) au moins un polymère consolidant et/ou filmogène dans une quantité de 1,0% en poids à 9,0% en poids par rapport au poids de l'agent, le polymère consolidant et/ou filmogène comportant au moins un motif structurel de la formule (I) et au moins un motif structurel de la formule (II)

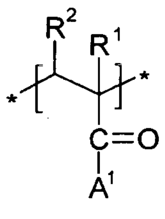 (I) 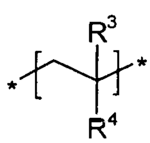 (II)

dans lesquelles

$R^1$ et $R^2$ représentent, indépendamment l'un de l'autre, un atome d'hydrogène ou un groupe méthyle, à condition que $R^1$ et $R^2$ ne représentent pas simultanément un groupe méthyle,
$R^3$ représente un atome d'hydrogène ou un groupe méthyle,
$R^4$ représente un groupe carbamoyle, un groupe alkylaminocarbonyle en $C_4$ à $C_{12}$ linéaire ou ramifié, un groupe alkylaminoéthylaminocarbonyle en $C_4$ à $C_{12}$ linéaire ou ramifié, un groupe alkylaminopropylaminocarbonyle en $C_4$ à $C_{12}$ linéaire ou ramifié, un groupe alkyloxycarbonyle en $C_4$ à $C_{12}$ linéaire ou ramifié, un groupe alkylaminoéthyloxycarbonyle en $C_4$ à $C_{12}$ linéaire ou ramifié, un groupe alkylaminopropyloxycarbonyle en $C_4$ à $C_{12}$ linéaire ou ramifié, un groupe alcyloxycarbonyle en $C_2$ à $C_{12}$ linéaire ou ramifié,
$A^1$ représente un groupe hydroxyle ou un radical organique comportant au moins un groupe acide sulfonique qui est lié par un atome d'oxygène ou un groupe NH au fragment structurel, pour améliorer le niveau de couleur des fibres de kératine colorées par oxydation, en particulier des cheveux humains colorés par oxydation.

2. Utilisation selon la revendication 1, **caractérisée en ce que** l'amidon non ionique modifié par de l'oxyde de propylène est un amidon de tapioca non ionique modifié par de l'oxyde de propylène ou un amidon de pomme de terre non ionique modifié par de l'oxyde de propylène ou un mélange des deux amidons susmentionnés.

3. Utilisation selon l'une des revendications précédentes, **caractérisée en ce que** l'agent contient l'amidon non ionique modifié par de l'oxyde de propylène dans une quantité de 8,0 à 12% en poids par rapport au poids de l'agent.

4. Utilisation selon l'une des revendications précédentes, **caractérisée en ce que** l'amidon non ionique modifié par de l'oxyde de propylène a une teneur en oxyde de propylène de 4 à 12% en poids, de manière particulièrement préférée une teneur en oxyde de propylène de 9,5 à 10,5% en poids ou de 4,0 à 6,0% en poids, sur la base du poids de l'amidon modifié.

5. Utilisation selon l'une des revendications précédentes, **caractérisée en ce qu'**au moins un amidon non ionique non réticulé modifié par de l'oxyde de propylène est contenu en tant qu'amidon non ionique modifié par l'oxyde de propylène.

6. Utilisation selon la revendication précédente, **caractérisée en ce que** le polymère anionique consolidant et/ou anionique filmogène (b) contient au moins un motif structurel de la formule (I) qui est choisi parmi au moins un motif structurel des formules (I-1) à (I-5)

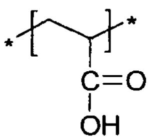
(I-1)
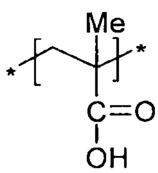
(I-2)
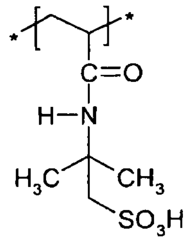
(I-3)

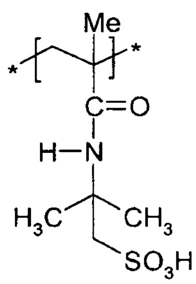
(I-4)
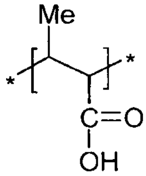
(I-5).

7. Utilisation selon l'une des revendications précédentes, **caractérisée en ce que** le polymère anionique consolidant et/ou anionique filmogène (b) contient au moins un motif structurel de la formule (II) qui est choisi parmi au moins

un motif structurel des formules (II-1) à (II-15)

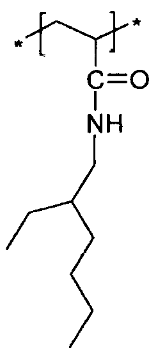
(II-1)

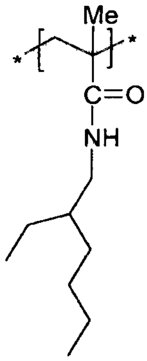
(II-2)

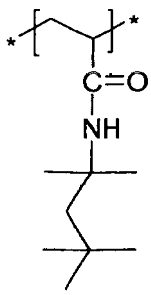
(II-3)

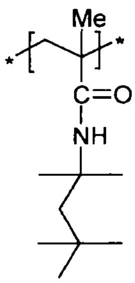
(II-4)

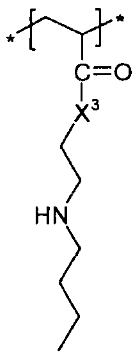
(II-5)

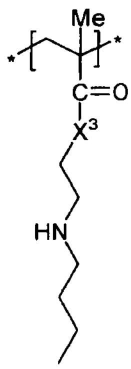
(II-6)

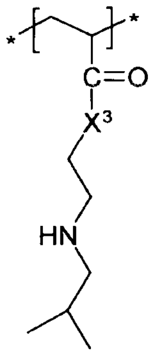
(II-7)

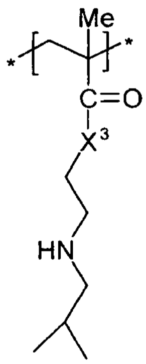
(II-8)

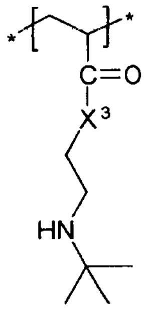
(II-9)

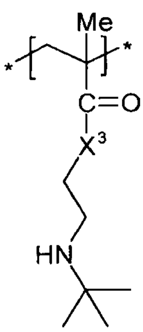
(II-10)

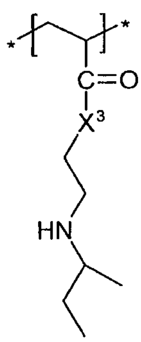
(II-11)

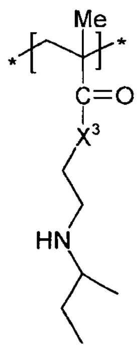
(II-12)

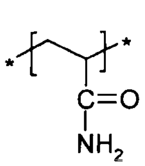 (II-13) 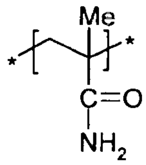 (II-14) 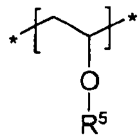 (II-15)

dans lesquelles

$X^3$ est un atome d'oxygène ou un groupe NH,
$R^5$ est un groupe acyle en $C_2$ à $C_{12}$ (en particulier l'acétyle ou le néodécanoyle).

**8.** Utilisation selon l'une des revendications précédentes, **caractérisée en ce que** le polymère anionique consolidant et/ou anionique filmogène (b) est choisi parmi au moins un polymère du groupe formé à partir de

- copolymères d'acide acrylique, d'acrylates d'alkyle en $C_1$ à $C_4$, de méthacrylate d'alkylaminoéthyle en $C_4$ et d'acrylamide d'alkyle en $C_{8,}$
- copolymères d'acide 2-acrylamido-2-méthyl-propanesulfonique et d'acrylamide,
- copolymères d'acide 2-acrylamido-2-méthyl-propanesulfonique, d'acrylamide et d'acide acrylique,
- copolymères d'acétate de vinyle et d'acide crotonique,
- copolymères de propionate de vinyle et d'acide crotonique,
- copolymères de néodécanoate de vinyle, d'acétate de vinyle et d'acide crotonique,
- copolymères d'acide méthacrylique et d'acrylate d'éthyle et d'acrylate de tert-butyle.

**9.** Utilisation selon l'une des revendications précédentes, **caractérisée en ce que** l'agent comprend les polymères anioniques consolidant et/ou anioniques filmogènes (b) dans une quantité de 3,0% en poids à 8,0% en poids, à chaque fois par rapport au poids de l'agent.

**10.** Utilisation selon l'une des revendications précédentes, **caractérisée en ce que** l'agent se présente sous la forme d'une mousse aérosol ou d'un spray aérosol.

**11.** Utilisation selon l'une des revendications précédentes, **caractérisée en ce que** l'agent a un pH (25°C) de 4,0 à 9,0, de préférence de 4,5 à 7,5 et en particulier de 5,0 à 6,0.

**12.** Utilisation selon l'une des revendications précédentes pour améliorer la solidité au lavage de fibres de kératine colorées par oxydation, en particulier de cheveux humains colorés par oxydation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1915981 B1
- EP 1923042 B1
- EP 1568351 A1
- FR 2944967 A1
- WO 20091345855 A2
- FR 2936416 A1
- DE OS19738866 A
- DE OS19736906 A
- DE OS19756454 A
- EP 998908 A2